# EUROPÄISCHE PATENTSCHRIFT

(11) **EP 4 387 568 B1**
(45) Veröffentlichungstag und Bekanntmachung des Hinweises auf die Patenterteilung: **18.02.2026**
(21) Anmeldenummer: 22761249.6
(22) Anmeldetag: 11.08.2022
(51) Int. Cl.: A61F 2/16

(54) **SYSTEM MIT EINEM INJEKTOR FÜR EINE INTRAOKULARLINSE**
SYSTEM WITH AN INJECTOR FOR AN INTRAOCULAR LENS
SYSTÈME AVEC UN INJECTEUR POUR LENTILLE INTRAOCULAIRE

(30) Priorität: 19.08.2021 DE 102021121575
(43) Veröffentlichungstag der Anmeldung: 26.06.2024
(73) Patentinhaber: Iolution GmbH, 22761 Hamburg (DE)
(72) Erfinder: MAROSCHECK, Christoph, 22761 Hamburg (DE); GUTIERREZ, Maximiliano, 22765 Hamburg (DE)
(74) Vertreter: Friderichs, Gunther
(86) Internationale Anmeldenummer: PCT/EP2022/072505
(87) Internationale Veröffentlichungsnummer: WO 2023/020923

(56) Entgegenhaltungen:
- WO-A1-2012/155887
- WO-A2-2018/006889

## Beschreibung

### Gebiet der Erfindung

Die Erfindung betrifft einen Injektor für eine Intraokularlinse. Weiter betrifft die Erfindung ein System mit einem Behälter zum Aufbewahren und Transportieren einer Intraokularlinse und einem Injektor für die Intraokularlinse.

### Hintergrund der Erfindung

Zum Applizieren einer künstlich hergestellten Intraokularlinse, beispielsweise im Rahmen einer Katarakt-Operation, sind Injektoren bekannt.

Es handelt sich dabei in der Regel um ein als Einweginstrument ausgebildetes Werkzeug, welches einen Kolben umfasst, mittels dessen die Linse vom Operateur aus einer Kanüle heraus ins Auge gedrückt werden kann.

Die aus einem Kunststoff ausgebildeten Intraokularlinsen können zum Applizieren gefaltet werden. So kann der Kanal, aus welchem die Linse herausgedrückt wird, einen kleineren Querschnitt aufweisen.

Weiter umfassen derartige Linsen in der Regel eine sogenannte Haptik. Dies sind federnd ausklappbare Arme, welche die Linse im Auge des Patienten zentrieren. Vorteilhafterweise wird die Haptik vor dem Falten der Linse eingefaltet.

Hierfür beschreibt die Offenlegungsschrift WO 2018/006889 A2 (Iolution GmbH) ein System mit einem Injektor und einem auf den Injektor aufschiebbaren Magazin, welches die Linse umfasst. Das Magazin umfasst einen Haptikschieber, welcher gemäß der Lehre vorstehend genannten Dokuments zwei seitlich angeordnete Schiebeelemente umfasst, über die der Benutzer zunächst die Haptik einfaltet, sodann einen Deckel mit einem Faltschwert zum Falten der Linse schließt. Die Linse ist nunmehr mit eingefalteten Armen gefaltet und kann sodann vom Operateur appliziert werden. Die Offenlegungsschrift WO 2012/155887 A1 zeigt einen Injektor mit einem Schieben zum Einfalten der Linsenhaptik.

### Aufgabe der Erfindung

Der Erfindung liegt gegenüber eingangs genanntem Stand der Technik die Aufgabe zugrunde, einen Injektor bzw. ein System mit einem Injektor für eine Intraokularlinse bereitzustellen, bei welchem das Betätigen des Schiebers für die Haptik der Intraokularlinse weiter vereinfacht ist.

### Zusammenfassung der Erfindung

Die Aufgabe der Erfindung wird bereits durch ein System mit einem Behälter und mit einem Injektor für eine Intraokularlinse nach Anspruch gelöst.

Bevorzugte Ausführungsformen und Weiterbildungen der Erfindung lassen sich dem Gegenstand der abhängigen Ansprüche, der Beschreibung sowie den Zeichnungen entnehmen.

Gemäß einem ersten Aspekt der Offenbarung bezieht sich diese auf einen Injektor für eine Intraokularlinse.

Der Injektor umfasst ein Gehäuse mit einem im Gehäuse verschiebbaren Kolben zum Applizieren der Intraokularlinse. Der Kolben ist mit einem Griff verbunden, über den der Benutzer den Kolben, bei einer Ausführungsform auch über einen Spindelantrieb, vorschieben kann und so über eine Kanüle ins Auge injizieren lassen kann.

Das Gehäuse kann mehrteilig aufgebaut sein. Insbesondere kann das Gehäuse einen ersten Abschnitt umfassen, welcher die Kanüle zum Applizieren der Linse umfasst und welcher mit einem Magazin, in welchem die Intraokularlinse angeordnet ist, verbunden werden kann. Ein zweiter Gehäuseabschnitt kann eine Führung für den Kolben nebst Kolbenstange beinhalten. Der Injektor ist vorzugsweise als Einweginstrument aus Kunststoff ausgebildet. Die Bestandteile des Injektors sind vorzugsweise ohne Verwendung eines Klebstoffes miteinander verbunden. Insbesondere können die Gehäuseabschnitte miteinander verrastet sein.

Gemäß der Erfindung umfasst der Injektor einen Haptikschieber, welcher durch das Aufschieben des Magazins auf das Gehäuse des Injektors aktivierbar ist.

Die Erfindung sieht also vor, dass der Haptikschieber automatisch durch das Aufschieben des Magazins auf den Injektor aktiviert wird.

Es ist insbesondere vorgesehen, dass das Magazin von der distalen Seite des Injektors in proximaler Richtung aufgeschoben wird, also entsprechend der Mittelachse des Kolbens. Gemäß einer anderen Ausführungsform kann das Magazin aber auch von oben, von der Seite oder von unten auf das Gehäuse des Injektors geschoben werden.

Insbesondere ist der Injektor derart ausgebildet, dass das Faltelement erst aktiviert werden kann, nachdem die Haptik mittels des Haptikschiebers eingefaltet wurde.

Der Haptikschieber ist vorzugsweise Teil des Magazins.

Es ist insbesondere vorgesehen, dass der Haptikschieber ein erstes Schiebeelement und ein zweites Schiebeelement umfasst.

Insbesondere können die Schiebeelemente in einer Ebene oberhalb oder unterhalb der Ebene, in welcher die Intraokularlinse im Magazin gehalten wird, angeordnet sein.

Die Schiebeelemente können insbesondere als seitlich angeordnete verschiebbare Schlitten ausgebildet sein, welche sich in entgegengesetzte Richtung bewegen und welche jeweils einen Arm der Haptik einklappen.

Vorzugsweise sind die Schiebeelemente miteinander gekoppelt, insbesondere mittels einer Wippe, welche gelenkig mit den Schiebeelementen verbunden ist.

Eines der Schiebeelemente bzw. ein mit dem Schiebeelement verbundenes Bauteil wird beim Aufschieben des Magazins dadurch bewegt, dass es an einen Anschlag des Gehäuses des Injektors stößt, also festgehalten wird.

Über eine drehbar gelagerte Wippe kann das gegenüberliegende Schiebeelement in die andere Richtung bewegt werden.

Die Haptiken werden so auf einfache Weise von beiden Seiten her in Richtung des optischen Teils der Intraokularlinse eingefaltet.

Gemäß einem weiteren Aspekt der Offenbarung bezieht sich die Erfindung auf einen Injektor für eine Intraokularlinse, insbesondere auf einen Injektor, wie dieser vorstehend beschrieben wurde.

Der Injektor umfasst ein Gehäuse mit einem im Gehäuse verschiebbaren Kolben zum Applizieren der Intraokularlinse. Weiter umfasst der Injektor ein aufschiebbares Magazin für die Intraokularlinse.

Der Injektor umfasst ebenfalls einen Haptikschieber zum Einfalten der Haptik der Intraokularlinse.

Weiter umfasst der Injektor ein Faltelement zum Falten der Intraokularlinse.

Wie eingangs beschrieben, dient das Faltelement dem Zusammenfalten der Intraokularlinse, so dass diese kompakter ausgebildet ist und so besser durch die Kanüle passt.

Das Faltelement kann insbesondere an einer Klappe angeordnet sein, welche auf das Magazin geklappt wird. Das Faltelement taucht beim Einklappen in das Magazin ein, faltet dabei die Intraokularlinse und transferiert diese aus dem Magazin in einen Kanal, durch welchen die Linse mittels des Kolbens aus dem Injektor herausgeschoben werden kann.

Gemäß der Erfindung ist das Faltelement erst nach einer Betätigung des Haptikschiebers zum Falten der Intraokularlinse freigegeben.

Hierunter wird verstanden, dass das Faltelement erst dann seine bestimmungsgemäße Funktion erfüllen kann, wenn die Haptik der Intraokularlinse bereits mittels des Haptikschiebers eingefaltet wurde.

Im nicht betätigten Zustand des Haptikschiebers ist dagegen das Faltelement derart blockiert oder gesperrt, dass dieses nicht in das Magazin eintauchen und die Intraokularlinse in das Gehäuse des Injektors transferieren kann.

Gemäß einer Ausführungsform der Erfindung blockiert der Haptikschieber oder ein mit dem Haptikschieber verbundenes Bauteil das Faltelement.

Insbesondere kann der Haptikschieber, sofern dieser nicht betätigt ist, als Anschlag für das als Klappe ausgebildete Faltelement dienen.

Gemäß einer Ausführungsform ist der Haptikschieber als Drehteller ausgebildet, welcher einen Durchlass für das Faltelement aufweist, wenn dieser in die betätigte Stellung gedreht wurde.

Im nicht betätigten Zustand steht der Durchlass für das Faltelement quer zum Faltelement. Ein Eintauchen des Faltelements in das Magazin wird so blockiert.

Gemäß einem weiteren Aspekt der Offenbarung bezieht sich dieser auf einen Injektor für eine Intraokularlinse, insbesondere einem der vorstehend beschriebenen Injektoren.

Dieser umfasst ein Gehäuse mit einem im Gehäuse verschiebbaren Kolben zum Applizieren der Intraokularlinse, auf aufschiebbares Magazin für die Intraokularlinse, ein Faltelement zum Falten der Intraokularlinse sowie einen Haptikschieber zum Einfalten der Haptik der Intraokularlinse.

Gemäß der Offenbarung umfasst der Haptikschieber ein erstes und ein zweites Schiebeelement, wobei erstes und zweites Schiebeelement derart ausgebildet sind, dass sich diese beim Einfalten der Haptik in dieselbe Richtung bewegen.

Im Unterschied zum eingangs beschriebenen Stand der Technik, bei welchem sich die Schiebeelemente in unterschiedliche Richtungen bewegen, ist bei dieser Ausführungsform lediglich eine einzige Bewegungsrichtung vorgesehen.

Dies kann beispielsweise die Rotation um eine Achse sein.

Insbesondere kann der Haptikschieber verdrehbar ausgebildet sein. Insbesondere wird der Haptikschieber durch einen Drehteller gebildet.

Diese Ausgestaltung erleichtert zum einen die Betätigung des Haptikschiebers, soweit dieser manuell betätigt wird.

Weiter kann so der Haptikschieber auf einfache Weise als Sperrelement ausgestaltet sein, welcher im nicht betätigten Zustand das Eintauchen des Faltschwertes blockiert.

In einer eingefalteten Stellung der Haptik umfasst der Haptikschieber dagegen einen Durchlass für das Faltelement.

Gemäß einem weiteren Aspekt betrifft die Offenbarung einen Injektor für eine Intraokularlinse, insbesondere einen Injektor, wie dieser vorstehend beschrieben wurde. Dieser umfasst ein Gehäuse mit einem im Gehäuse verschiebbaren Kolben zum Applizieren der Intraokularlinse, ein auf das Gehäuse des Injektors aufschiebbares Magazin für die Intraokularlinse, ein Faltelement zum Falten der Intraokularlinse sowie einen Haptikschieber zum Einfalten der Haptik der Intraokularlinse.

Gemäß der Erfindung umfasst der Haptikschieber ein erstes und ein zweites Schiebeelement, wobei erstes und zweites Schiebeelement zum Einfalten der Haptik aufeinander zu bewegbar sind.

Im Unterschied zum eingangs beschriebenen Stand der Technik, bei dem sich die Schiebeelemente parallel zueinander und in verschiedene Richtungen bewegen, ist also vorgesehen, dass sich die Schiebeelemente aufeinander zubewegen.

Bei einer manuellen Betätigung des Haptikschiebers erleichtert dies das Aktivieren des Haptikschiebers. Insbesondere kann beispielsweise das Magazin mit zwei Fingern gefasst werden. Beim Zusammenpressen bewegen sich die beiden Schiebeelemente aufeinander zu und falten die gegenüberliegenden Arme der Haptik ein.

Dies kann insbesondere durch seitlich, also links und rechts am Gehäuse angeordnete Schiebeelemente realisiert sein.

Gemäß einer weiteren Ausführungsform werden die Schiebeelemente in axialer Richtung bezüglich der Mittelachse des Kolbens aufeinander zubewegt.

Es ist insbesondere vorgesehen, dass der Injektor, insbesondere das Magazin des Injektors, teleskopartig ausgebildet ist. Die Intraokularlinse kann dabei in einem Mittelteil gehalten sein. Ein distales Schiebeelement faltet einen Arm der Haptik der Intraokularlinse ein. Das Mittelteil schiebt sich in ein proximales Schiebeelement bzw. das proximale Schiebeelement bewegt sich aufgrund des Einschiebens des Mittelteils relativ auf das distale Schiebeelement zu und faltet einen gegenüberliegenden Arm der Haptik ein.

Diese Ausführungsform der Erfindung eignet sich insbesondere für ein automatisiertes Einfalten der Haptiken.

Insbesondere kann beim Aufschieben des Magazins das distale Schiebeelement gefasst werden. Beim Aufschieben des Magazins auf das Gehäuse des Injektors verbindet sich das Magazin mit dem Gehäuse des Injektors und wird gleichzeitig derart zusammengeschoben, dass sich distales und proximales Schiebeelement aufeinander zubewegen.

Weiter ist ein automatisiertes Aktivieren des Haptikschiebers auch dadurch möglich, dass das distale Schiebeelement, beispielsweise in einem Wasserbehälter, in welchem das Magazin mit der Intraokularlinse gelagert ist, gegen einen Anschlag des Behälters stößt, wenn das Gehäuse des Injektors in den Behälter eingetaucht wird, um das Magazin mit dem Gehäuse zu verbinden.

Gemäß einem weiteren Aspekt der Offenbarung betrifft dieser einen Injektor für eine Intraokularlinse, insbesondere einen Injektor, wie dieser vorstehend beschrieben wurde.

Der Injektor umfasst ein Gehäuse mit einem im Gehäuse verschiebbaren Kolben zum Applizieren der Intraokularlinse, ein auf das Gehäuse aufschiebbares Magazin für bzw. mit der Intraokularlinse, ein Faltelement zum Falten der Intraokularlinse sowie einen Haptikschieber zum Einfalten der Haptik der Intraokularlinse.

Gemäß der Erfindung ist der Haptikschieber auf einer Unterseite des Magazins angeordnet. Unter der Unterseite des Magazins wird diejenige Seite verstanden, welche bei bestimmungsgemäßer Verwendung des Magazins an das Gehäuse des Injektors angrenzt. Das Faltelement kann dagegen durch die Oberseite des Magazins eintauchen.

Die erfindungsgemäße Anordnung auf der Unterseite, insbesondere in einer Ebene, welche unter der Ebene liegt, in welcher die Intraokularlinse im Magazin gehalten ist, ermöglicht zum einen eine besonders manipulationssichere Ausgestaltung des Haptikschiebers.

Bei dieser Ausführungsform der Erfindung kann der Haptikschieber insbesondere zwei seitlich gegenüberliegend angeordnete Schiebeelemente umfassen.

Diese werden zum Einfalten der Arme der Haptik in entgegengesetzter Richtung zueinander bewegt.

Gemäß einer bevorzugten Ausführungsform wird nur eines der Schiebeelemente relativ zum Gehäuse bewegt. Bei dieser Ausführungsform der Erfindung wird vielmehr die Linse selbst über das erste Schiebeelement bewegt. Insbesondere kann die Linse durch eine Rotationsbewegung in Richtung des zweiten Schiebeelements bewegt werden, welches als Anschlag für einen Arm der Haptik ausgebildet ist und so den dem ersten Schiebeelement gegenüberliegenden Arm der Haptik einfaltet.

Die Erfindung betrifft ein System mit einem Injektor für eine Intraokularlinse, insbesondere einem Injektor, wie dieser vorstehend beschrieben wurde.

Der Injektor umfasst einen Haptikschieber zum Einfalten der Haptik der Intraokularlinse. Weiter umfasst das System einen Behälter.

Bei dem Behälter kann es sich insbesondere um einen mit Flüssigkeit gefüllten Behälter zum Lagern und Transportieren einer hydrophilen Intraokularlinse handeln.

Weiter kann es sich bei dem Behälter um einen Wärmebehälter handeln, der dazu ausgebildet ist, die in dem Magazin befindliche Linse vor dem Applizieren zu erwärmen, damit diese flexibler ist und sich leichter falten lässt.

Gemäß der Erfindung ist der Haptikschieber durch das Einbringen des Injektors in den Behälter aktivierbar.

Im Falle der Verwendung eines Wärmebehälters kann beispielsweise der Haptikschieber durch Einbringen des Magazins nebst Injektor aktivierbar sein.

Weiter kann das Magazin in dem Behälter in einem hierfür ausgebildeten Gehäuseabschnitt, insbesondere einem Kanal angeordnet sein.

Das System ist dabei derart ausgebildet, dass das Magazin mittels des Gehäuses des Injektors aufgenommen werden kann.

Beim Koppeln des Magazins mit dem Injektor wird automatisch der Haptikschieber aktiviert. Dies kann beispielsweise dadurch erfolgen, dass das Magazin und/oder das Gehäuse des Injektors auf einen Anschlag gedrückt wird, der den Haptikschieber aktiviert.

So lässt sich ebenfalls eine besonders einfache Handhabung des Systems mit dem Injektor realisieren. Weiter kann das Faltelement, wie vorstehend ausgeführt, gesperrt sein, bis der Haptikschieber durch das Koppeln aktiviert wurde.

Der Behälter kann eine Aufnahme für das Magazin und/oder den Injektor umfassen. Weiter kann der Behälter einen Anschlag für das Magazin und/oder den Injektor umfassen, der derart ausgebildet ist, dass er den Haptikschieber aktiviert, wenn der Injektor in den Behälter geschoben wird.

Bei einer Ausführungsform der Erfindung befindet sich das Magazin in der Aufnahme des Behälters. Der Haptikschieber wird aktiviert, wenn der Injektor in den Behälter geschoben wird und dabei mit dem Magazin gekoppelt wird.

### Kurzbeschreibung der Zeichnungen

Der Gegenstand der Erfindung soll im Folgenden bezugnehmend auf verschiedene Ausführungsbeispiele näher erläutert werden.
Fig. 1 bis Fig. 3 zeigen ein erstes Ausführungsbeispiel eines Injektors, bei welchem der Haptikschieber beim Entnehmen des Magazins aus einem Behälter automatisch aktiviert wird.
Fig. 4 zeigt das in dem Behälter angeordnete Magazin.
Fig. 5 bis Fig. 7 zeigen eine Ausführungsform eines Magazins, bei welchem der Haptikschieber beim Aufschieben auf das Gehäuse des Injektors automatisch aktiviert wird.
Fig. 7 ist dabei ein Längsschnitt entlang einer Horizontalebene.
Fig. 8 bis Fig. 10 zeigen einen Injektor, bei welchem das Magazin einen Haptikschieber mit zwei Schiebeelementen umfasst, welche axial aufeinander zu bewegbar sind.
Fig. 11 ist eine schematische Darstellung eines Injektors mit axial aufeinander zu bewegbaren Schiebeelementen.
Fig. 12 ist eine schematische Darstellung, bei welcher aufeinander zu bewegbare Schiebeelemente, welche gleichzeitig als Griffstück dienen, seitlich am Magazin angeordnet sind.
Fig. 13 bis Fig. 18 sind schematische Darstellungen eines Injektors mit einem als Drehteller ausgebildeten Haptikschieber.
Fig. 19 bis Fig. 21 sind schematische Darstellungen eines Injektors, bei welchem sich der Haptikschieber auf der Unterseite des Magazins befindet.

### Detaillierte Beschreibung der Zeichnungen

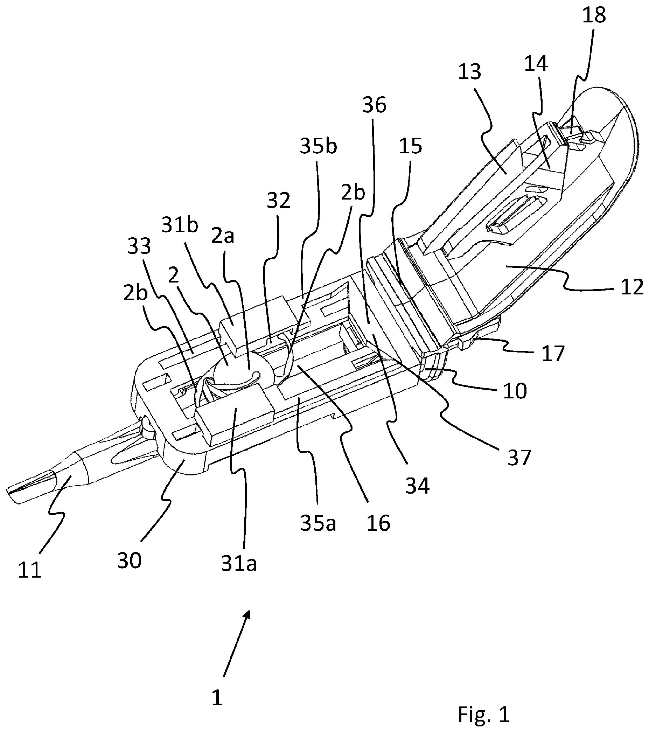

Fig. 1 ist eine perspektivische Ansicht eines Injektors 1, welcher derart ausgebildet ist, dass der Haptikschieber 31a, 31b beim Aufnehmen des Magazins 30 aus einem Behälter automatisch aktiviert wird.

In der perspektivischen Darstellung dargestellt ist das Gehäuse 10 des Injektors, wobei nur der vordere Gehäuseteil dargestellt ist, welches über Rasthaken 17 mit einem hinteren Gehäuseteil verbindbar ist.

Das hintere Gehäuseteil einschließlich Griffstück, Kolbenstange und Betätigungsorgan ist nicht dargestellt. Dieses kann entsprechend eingangs beschriebenem Stand der Technik ausgebildet sein.

Das Magazin 30 kann auf das Gehäuse 10 aufgeschoben werden.

In dem Magazin 30 ist die Intraokularlinse 2 angeordnet. Die Intraokularlinse 2 umfasst einen Linsenkörper 2a als optischen Bestandteil der Linse sowie die Haptik 2b, welche in diesem Ausführungsbeispiel zwei nach außen federnde Arme umfasst.

An dem Gehäuse 10 angeordnet ist die Klappe 12. Diese umfasst ein Faltelement 13, welches schwertartig ausgebildet ist und beweglich in dem Lagerbock 14 angeordnet ist.

Über dem Filmscharnier 15 kann das Faltelement 13 in das Magazin 30 eingeführt werden, wobei es die Intraokularlinse 2 faltet und in den Kanal 16 des Gehäuses 10 überführt, von wo aus die gefaltete Intraokularlinse 2 mittels des nicht dargestellten Kolbens aus der Kanüle 11 heraus appliziert werden kann.

Die Klappe 12 umfasst einen Rasthaken 18, welcher die Klappe 12 nach dem Herunterklappen am Magazin 30 verrastet. Hierdurch erhöht sich die Manipulationssicherheit des als Einwegsystem ausgebildeten Injektorsystems.

Bei einer weiteren Ausführungsform kann die Klappe auch an zumindest einer Seitenwand des Magazins verrasten (nicht dargestellt).

Zum Einfalten der Haptik 2a, 2b dienen die seitlich angeordneten Schiebeelemente 31a, 31b, welche entlang von Schienen, die beispielsweise als Nut 33 ausgebildet sind, gegeneinander verschiebbar sind.

Dargestellt ist in dieser Ansicht der nicht eingefaltete Zustand.

Die Faltelemente 31a, 31b umfassen ein Gehäuse mit jeweils einer Ausnehmung 32, in welcher das Ende des jeweiligen Arms der Haptik 2b sitzt. Über eine proximale Verschiebung des Schiebeelements 31a und eine distale Verschiebung des Schiebelements 31b werden die Arme der Haptik 2b über bzw. an den optischen Teil 2a der Intraokularlinse 2 gefaltet, bevor sodann die Linse 2 mittels des Faltelements 13 zusammengefaltet und in den Kanal 16 transferiert wird.

Die Faltelemente 31a, 31b sind über in Längsrichtung verlaufende die Stangen 35a, 35b mit einer Wippe 34 verbunden.

Die Wippe 34 ist auf der Drehachse 36 schwenkbar gelagert. Die Wippe 34 ist ihrerseits über die als Filmscharniere ausgebildeten Scharniere 37 mit den Stangen 35a, 35b verbunden. Sobald das erste Schiebeelement 31a an einen Anschlag stößt, verschiebt sich dieses in proximaler Richtung, wodurch über die Stange 35a die Wippe 34 bewegt wird und so auf der gegenüberliegenden Seite über die Stange 35b das Schiebeelement 31b in distaler Richtung bewegt.

Auf diese Art und Weise genügt es, auf eines der Schiebeelemente 31a in einer einzigen Richtung Kraft auszuüben, um beide Schiebeelemente 31a, 31b in gegenläufiger Richtung zu bewegen.

Bei einer weiteren, hier nicht dargestellten Ausführungsform, sind die Schiebelemente 31a und 31b nicht miteinander gekoppelt. Das Schiebelement 31a kann dabei z.B. durch das Aufnehmen des Injektors aus einem Behälter aktiviert werden (durch nach vorne Drücken). Das Schiebelement 31b kann bei Herausziehen des Injektors einschließlich des Magazins aus dem Behälter aktiviert werden, z.B. durch einen Hinterschnitt im Behälter. Ansonsten kann diese Ausführungsform wie hier in Fig. 1 bis Fig. 3 dargestellt ausgebildet sein.

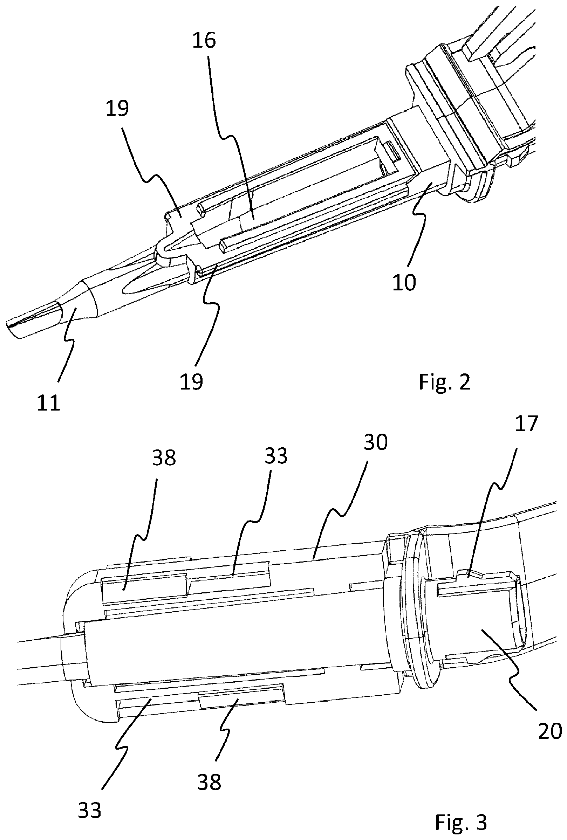

Fig. 2 ist eine Darstellung des Gehäuses 10 mit abgenommenem Magazin.

Zu erkennen ist eine Schiene 19, auf welche das Magazin aufschiebbar ist.

Im Bereich des Magazins ist das Gehäuse 10 oben offen und der Kanal 16, über den die Linse in die Kanüle 11 transferiert wird, offen.

Fig. 3 ist eine perspektivische Ansicht auf die Unterseite des in Fig. 1 und Fig. 2 dargestellten Injektors.

Das Magazin 30 ist nunmehr wieder aufgeschoben.

Auf der Unterseite des Magazins 30 ist zu erkennen, dass die Rasthaken 38 durch die Nuten 33, welche als Durchgangsnuten ausgebildet sind, des Gehäuses des Magazins 30 greifen. Dies ermöglicht eine leichte Montage des Magazins.

In proximaler Richtung nach dem Magazin 30 angeordnet, ist das Anschlussstück 20 mit den Rasthaken 17, über das der hier dargestellte Gehäuseabschnitt mit einem hinteren Gehäuseabschnitt verbunden wird.

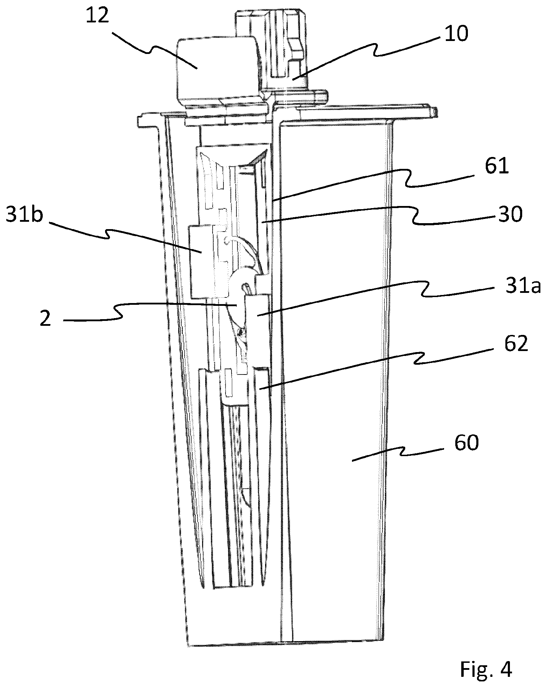

Fig. 4 zeigt schematisch, wie das Magazin 30 in einem Behälter 60 sitzt und mittels des Gehäuses 10 des Injektors aufgenommen wird.

Die Intraokularlinse 2 befindet sich vorgeladen im Magazin 30.

Das Magazin 30 ist in diesem Ausführungsbeispiel in einem Behälter 60 angeordnet, welcher mit Wasser befüllt ist. Vorzugsweise wird dieses System für hydrophile Intraokularlinsen 2 verwendet.

Der Behälter 60 umfasst einen Kanal 61, welcher einen an das Magazin 30 angepassten Aufnahmebereich bereitstellt.

Nach dem Öffnen des Behälters 60 wird der Injektor voran mit dem Gehäuse 10 in den Behälter eingetaucht und mit dem Magazin 30 verbunden.

Dabei wird das Magazin 30 mit dem ersten Haptikschieber 31a voran auf einen Anschlag 62 gedrückt.

Während des Verbindens wird so das Magazin 30 tiefer in den Behälter 60 hineingeschoben und es werden dabei die Haptikschieber 31a und 31b bewegt, so dass die Arme der Haptik auf den optischen Teil der Intraokularlinse 2 eingefaltet werden.

Das Magazin 30 verrastet vorzugsweise untrennbar mit dem Gehäuse 10 des Injektors und kann sodann zusammen mit der Intraokularlinse entnommen werden.

Der Benutzer braucht nunmehr nur noch die Klappe 12 einzuklappen, um die Intraokularlinse 2 in den zur Kanüle führenden Kanal zu transferieren.

Der Injektor ist nunmehr einsatzbereit und kann unmittelbar zum Applizieren der Linse ins Auge verwendet werden.

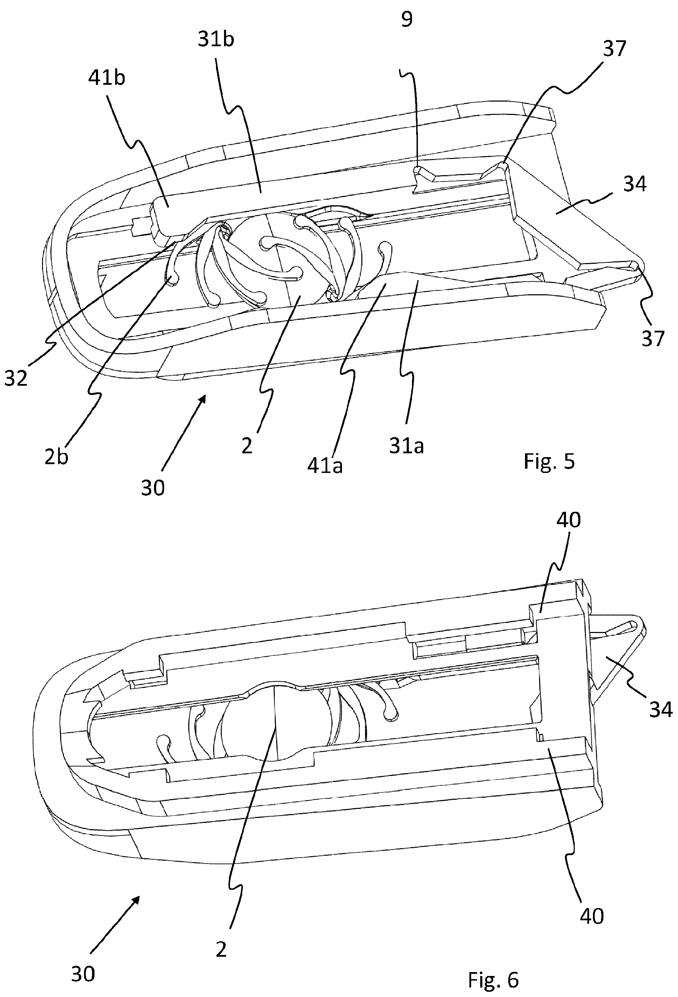

Fig. 5 ist eine perspektivische Ansicht eines Magazins 30, welches derart ausgebildet ist, dass sich der Haptikschieber 31a, 31b automatisch aktiviert, wenn das Magazin 30 auf den Injektor aufgeschoben wird.

Der Haptikschieber umfasst in diesem Ausführungsbeispiel an den gegenüberliegenden Seiten in Längsrichtung verschiebbare Schiebeelemente 31a, 31b, welche wie bei der in Fig. 1 bis Fig. 4 dargestellten Ausführungsform auf einer Schiene, wie beispielsweiser einer Nut, gelagert sind.

Weiter umfassen die Schiebeelemente 31a, 31b ebenfalls jeweils eine Ausnehmung 32, in welche der ausgeklappte Arm der Haptik 2b hineinragt.

Die Ausnehmungen 32 befinden sich bei diesem Ausführungsbeispiel in einem Kopf 41a, 41b, welcher gegenüber dem angrenzenden Teil des jeweiligen Schiebeelements 31a, 31b verdickt ausgebildet ist. Eine Anlagefläche innerhalb der Ausnehmung 32 für die Haptik 2b kann so schräg ausgerichtet sein, was das Einfalten der Haptik 2b erleichtert.

Die Schiebeelemente 31a, 31b sind auch bei diesem Ausführungsbeispiel mit einer drehbar gelagerten Wippe 34 verbunden.

Die Wippe 34a ist über ein Scharnier 37 (z.B. Filmscharnier) mit den Schiebeelementen 31a, 31b verbunden.

Im Unterschied zu dem zuvor dargestellten Ausführungsbeispiel ist noch ein weiteres Scharnier 39 vorhanden.

Durch das so zwischen dem ersten Scharnier 37 und dem zweiten Scharnier 39 vorhandene Zwischenstück kann kompensiert werden, dass sich die Erstreckung der Wippe 34 in Querrichtung mit zunehmender Auslenkung der Wippe 34 verringert.

Dargestellt in dieser Ansicht ist der Zustand bei nicht aktiviertem Haptikschieber. Die Bewegung der Arme der Haptik 2a ist schematisch durch die drei überlagerten Positionen dargstellt.

Das Magazin 30 ist derart ausgebildet, dass der Haptikschieber durch das Aufschieben des Magazins 30 auf das Gehäuse des Injektors aktiviert wird.

Dies ist bei diesem Ausführungsbeispiel dadurch realisiert, dass die Wippe 34 in dem hier dargestellten nicht aktivierten Zustand quer steht und so aus dem Grundgehäuse des Magazins 30 herausragt.

Beim Aufschieben auf das Gehäuse des Injektors stößt die Wippe 34 am Gehäuse des Injektors an.

Das Gehäuse des Injektors wirkt also als Anschlag, der bewirkt, dass sich die Wippe 34 geraderichtet.

Das Schiebeelement 31b wird dabei in proximaler Richtung und das Schiebeelement 31a in distaler Richtung bewegt. Dadurch werden die Arme der Haptik 2b in Richtung des optischen Teils der Linse 2 eingefaltet.

In Fig. 5 dargestellt ist die Oberseite des Magazins 30. Die Schiebeelemente 31a, 31b bewegen sich also auch bei dieser Ausführungsform auf einer Ebene, welche oberhalb der Ebene angeordnet ist, in welcher die Linse 2 im Magazin gehalten ist. Hier werden die Haptiken hier auf der Seite der Haptik-Optik Anbindung bewegt, in Fig. 1-3 an der geegnüberliegenden Seite.

Fig. 6 ist eine perspektivische Ansicht der Unterseite des Magazins 30.

Hier umfasst das Magazin gegenüberliegende Schienen 40 zum Aufschieben auf das Gehäuse des Injektors. Zu erkennen ist, dass die Wippe 34 auf der proximalen Seite des Magazins 30 herausragt.

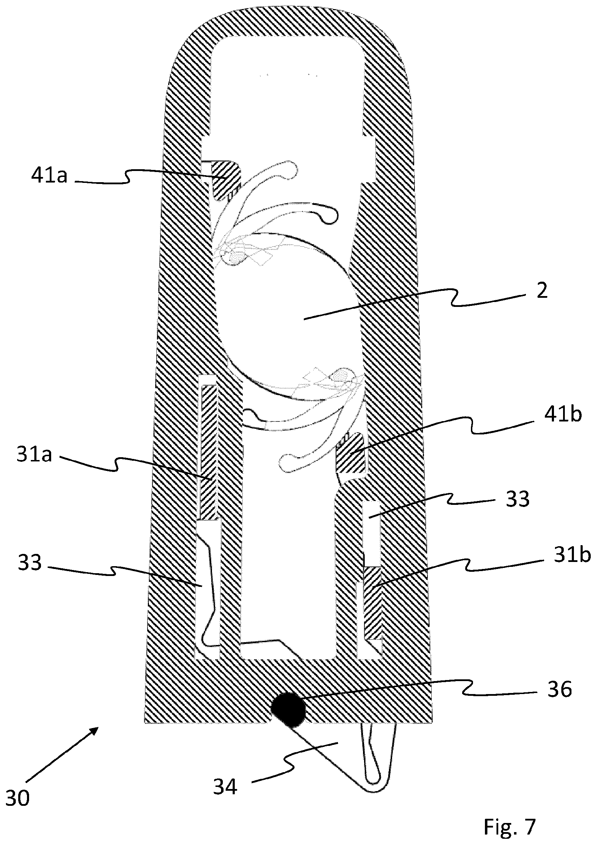

Fig. 7 ist ein Längsschnitt entlang einer Horizontalebene. Zu erkennen ist, dass die Schiebeelemente 31a, 31b jeweils durch eine Nut des Gehäuses des Magazins 30 greifen. Diese sind also in gleicher Weise wie bei der in Fig. 3 dargestellten Ausführungsform geführt. Allerdings greifen bei dieser Ausführungsform die Schiebelemente 31a, 31b nur in einem proximalen Bereich des Magazins durch die Nuten 33. In distaler Richtung verlaufen sodann die Schiebeelemente 31a, 31b auf der Oberseite des Magazins bis hin zum Kopf 41a, 41b, welcher nach unten in den Bereich, in dem die Linse 2 angeordnet ist, hineinragt.

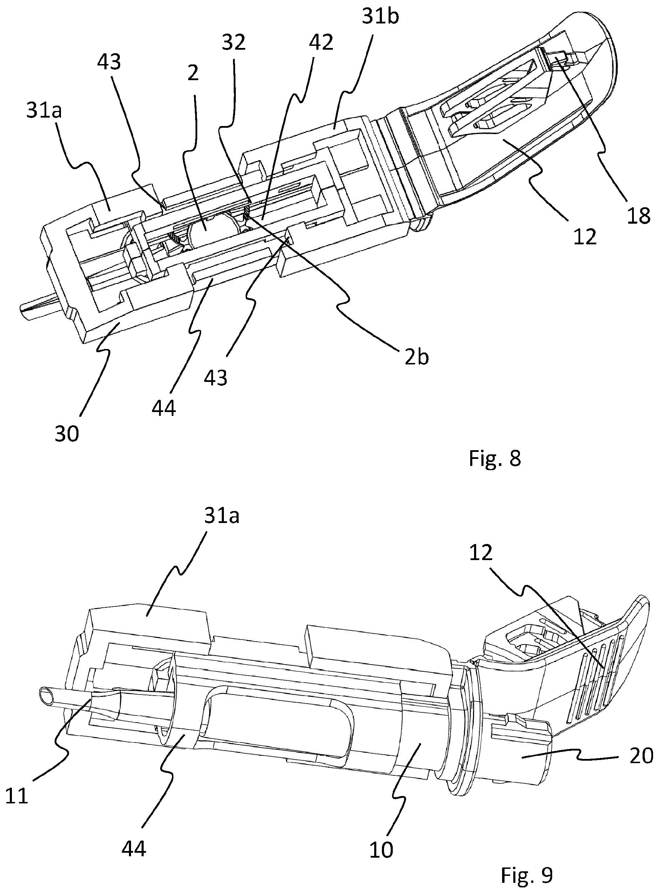

Fig. 8 ist eine Ausführungsform, bei welcher der Haptikschieber 2 gegenüberliegende Schiebeelemente 31a, 31b umfasst, die in axialer Richtung aufeinander zu bewegbar sind. Der Injektor bzw. das Magazin 30 des Injektors umfasst ein Mittelteil 44, welches über eine Schienenverbindung mit den Schienen 43 gegenüber den Schiebeelementen 31a, 31b verschiebbar ist.

Dargestellt ist der nicht aktivierte Zustand.

Die Schiebeelemente 31a, 31b umfassen jeweils zumindest eine Ausnehmung 32, in welche die Arme 2b der Haptik hineinragen.

Wird nun eine Kraft auf das distale Schiebeelement 31a ausgeübt (entweder manuell oder durch Auflaufen des Schiebeelements 31a auf einen Anschlag in einem Behälter, entsprechend Fig. 4), so schieben sich die Schiebeelemente 31a, 31b und das Mittelteil 44 teleskopartig zusammen.

Dabei wird die Haptik 2b der Linse 2 eingefaltet. Dabei wird zuerst der distale Arm der Haptik durch das Schiebelement 31a eingefaltet. Sodann schiebt sich das Mittelteil 44 in proximale Richtung und der proximale Arm der Haptik 2b ist eingefaltet, wenn das Schiebeelement 31a an dem Schiebelement 31b zum Anschlag kommt.

Bei dieser Ausführungsform kann der Injektor derart ausgebildet sein, dass in dem hier dargestellten nicht aktivierten Zustand die Klappe 12 mit dem Faltelement nicht geschlossen werden kann, da diese nicht in die Halteebene 42 für die Linse 2 eintauchen kann. Das Mittelteil 44 ist nämlich noch nicht eingeschoben.

Erst mit Zusammenschieben der Schiebeelemente 31a und 31b kann die Klappe 12 geschlossen werden und der Rasthaken 18 verrastet die Klappe 12.

Schiebeelemente 31a, 31b sowie das Mittelteil 44 verrasten vorzugsweise in der vollständig zusammengeschobenen Position.

Diese Ausführungsform ermöglicht das Schieben von je einer Haptik distal und proximal oder auch je nach Ausgestaltung der Lisenform von je 2 Haptiken distal wie proximal.

Fig. 9 ist eine perspektivische Ansicht der Unterseite des Injektors.

Das distale Schiebeelement 31a ragt im nicht aktivierten Zustand über das Mittelteil 44 und befindet sich oberhalb der Kanüle 11.

Das Mittelteil 44 umgreift die Kanüle 11.

Das Gehäuse 10 ist auch in diesem Ausführungsbeispiel mehrteilig aufgebaut. Der hier dargestellte vordere Gehäuseabschnitt umfasst ein Anschlussstück 20 für einen hinteren Gehäuseabschnitt.

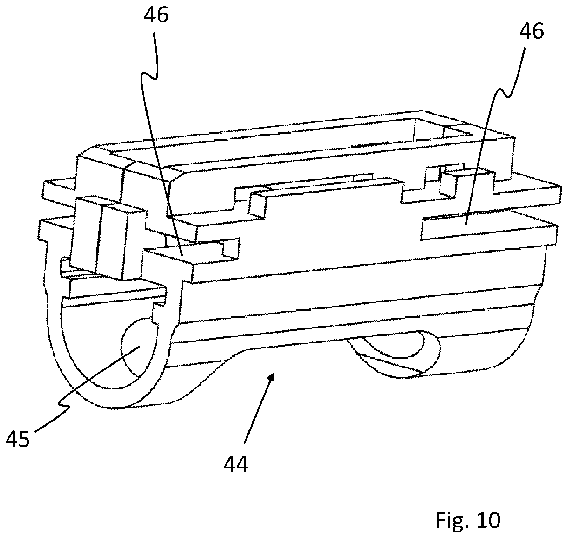

Fig. 10 ist eine perspektivische Ansicht des Mittelteils 44. Dieses umfasst einen Kanal 45, durch welchen im montierten Zustand die Kanüle mit dem Kanal, in den die gefaltete Linse transferiert wird, verläuft.

Weiter umfasst das Mittelteil 44 Schienen 46, mit denen dieses beweglich mit den Schiebeelementen verbunden wird.

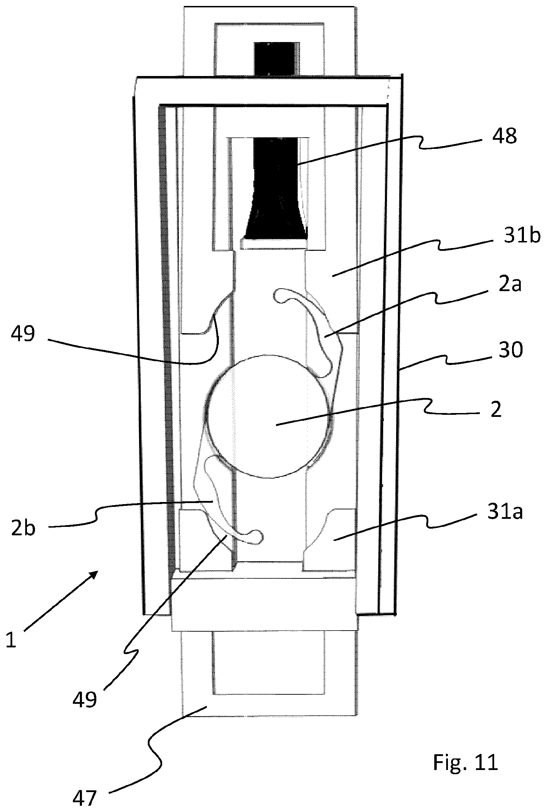

Fig. 11 ist eine weitere schematische Darstellung eines Injektors 1, welche axial aufeinander zu bewegbare Schiebeelemente 31a, 31b umfasst.

Das proximale Schiebeelement 31b ist mit dem Injektorgehäuse verbunden.

Das distale Schiebeelement 31a kann über das Kopfstück 47 in proximaler Richtung verschoben werden.

Die Schiebeelemente 31a, 31b umfassen jeweils gekrümmte Anlageflächen 49, welche das Einfalten der Haptik 2b erleichtern.

Das proximale Schiebeelement 31b wird in Richtung der Linse 2 entweder manuell oder durch Aufschieben des Magazins 30 auf das Injektorgehäuse bewegt..

Sobald die Haptik 2b eingefaltet ist, kann auch bei diesem Ausführungsbeispiel eine Klappe mit einem Faltelement geschlossen werden, wodurch die Linse gefaltet in einen unterhalb des Magazins angeordneten Kanal transferiert wird. Sodann kann die Linse 2 mittels des Kolbens 48 mit einem vorzugsweise weichen Endstück (z.B. aus Silikon) aus der in dieser schematischen Darstellung nicht dargestellten Kanüle herausgedrückt und so appliziert werden.

Denkbar ist sowohl einer Variante, bei welcher die Schiebeelemente 31a, 31b manuell, z.B. beim Herausnehmen aus einem Behälter aktiviert werden, als auch eine Variante, bei der die Schiebeelemente 31a, 31b mit dem Aufschieben des Magazin 30 auf das Injektorgehäuse eingeschoben werden.

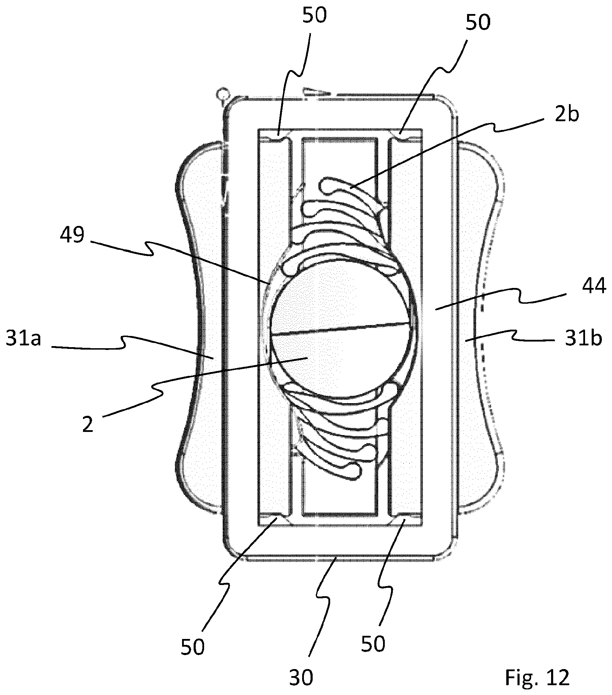

Fig. 12 ist eine schematische Ansicht eines Ausführungsbeispiels, bei welchem die Schiebeelemente 31a, 31b seitlich aus dem Gehäuse des Magazins herausragen.

Zum Einfalten der Haptik 2b umfassen die Schiebeelemente 31a, 31b jeweils eine gekrümmte Anlagefläche 49.

Die Schiebeelemente 31a, 31b sind als Griffstück ausgebildet und können eine gekrümmte Außenseite aufweisen.

Dargestellt ist der nicht aktivierte Zustand. Die Bewegung der Arme der Haptik 2b ist aber wiederum durch überlagerte Positionen schematisch dargestellt.

Der Benutzer kann das Magazin 30 an den seitlichen Schiebeelementen 31a, 31b greifen. Presst nunmehr der Benutzer die Schiebeelemente 31a, 31b zusammen, so bewegen sich diese aufeinander zu und falten die Haptik 2b ein.

Im eingefalteten Zustand verrasten die Schiebeelemente 31a, 31b mittels der Rasthaken 50 am Gehäuse des Magazins 30.

Diese Ausführungsform ermöglicht eine sehr einfache manuelle Aktivierung des Haptikschiebers. Dieser kann quasi "on the fly" aktiviert werden, wenn der Benutzer das Magazin 30 greift, um auf das Gehäuse des Injektors aufzuschieben.

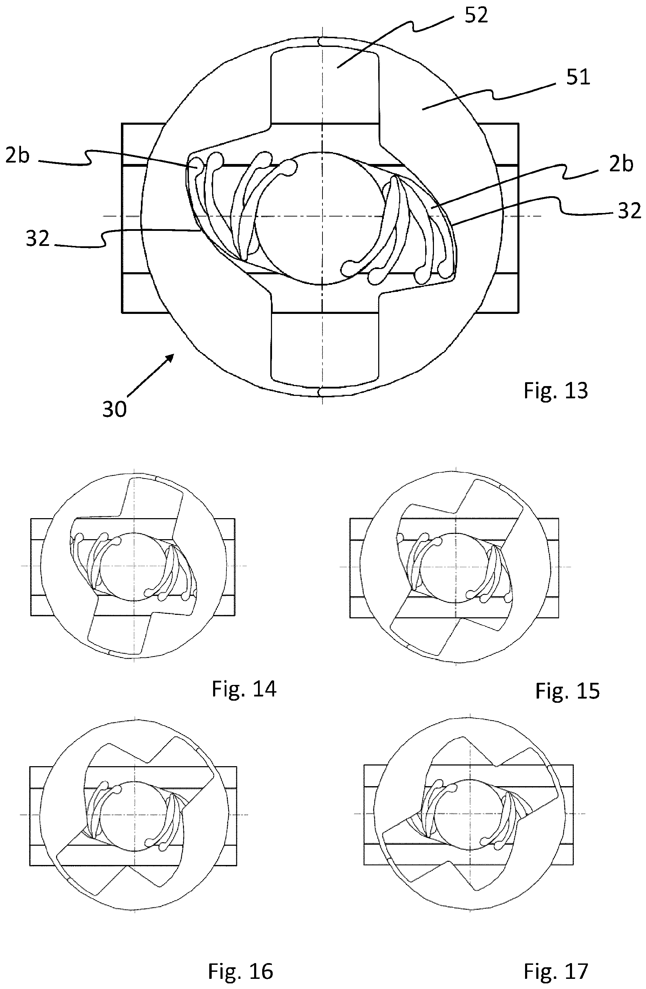

Fig. 13 bis Fig. 18 zeigen in einer schematischen Darstellung ein Magazin 30 mit einem Haptikschieber, der als Drehteller 51 ausgebildet ist.

Der Drehteller 51 ist drehbar auf dem Gehäuse des Magazins 30 gelagert.

Fig. 13 zeigt in einer Draufsicht von oben den nicht aktivierten Zustand.

Der Drehteller 51 umfasst Ausnehmungen 32, welche gekrümmt ausgebildet sind.

In dem hier dargestellten nicht aktivierten Zustand können die Arme der Haptik 2b in die Ausnehmungen 32 einfedern.

Weiter umfasst der Drehteller 51 einen Durchlass 52 für das Faltelement.

Der Durchlass 52 für das Faltelement ist in dem hier dargestellten nicht aktivierten Zustand quer zum Faltelement ausgerichtet.

Da die Ausnehmungen 32 nicht genug Raum für das Faltelement freigeben, ist in dem nicht aktivierten Zustand ein Eintauchen des Faltelements in das Gehäuse des Magazins gesperrt. Dieses kommt am Drehteller 51 zur Anlage.

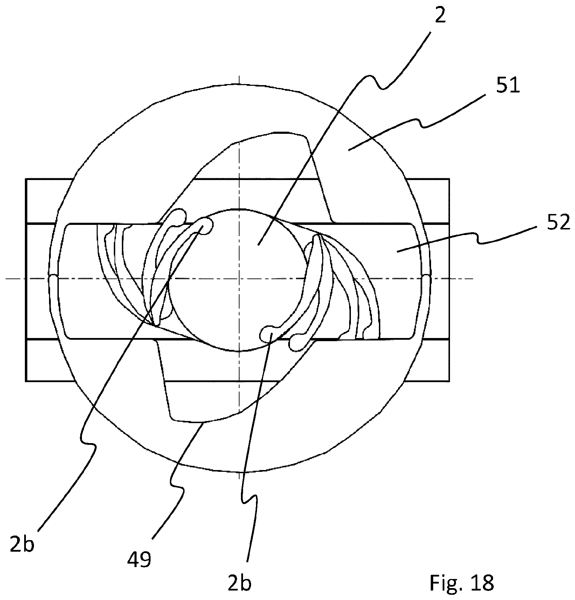

In den Figuren Fig. 14 bis Fig. 18 ist dargestellt, wie nunmehr der Benutzer den Drehteller 51 um eine Viertelumdrehung dreht, bis dieser die in Fig. 18 dargestellte Endposition erreicht. Durch das Drehen klappen die Anlageflächen 49 die Arme der Haptik 2b ein.

Der Durchlass 52 ist nunmehr in der Endposition in Längsrichtung ausgerichtet und das Faltelement kann nunmehr durch den Durchlass 52 eintauchen, die Linse 2 falten und in den darunterliegenden Kanal, welcher zur Kanüle führt, transferieren.

Vorzugsweise ist der Drehteller 51 zumindest in der Endposition gegen ein Zurückdrehen gesichert (beispielsweise über eine Verrastung).

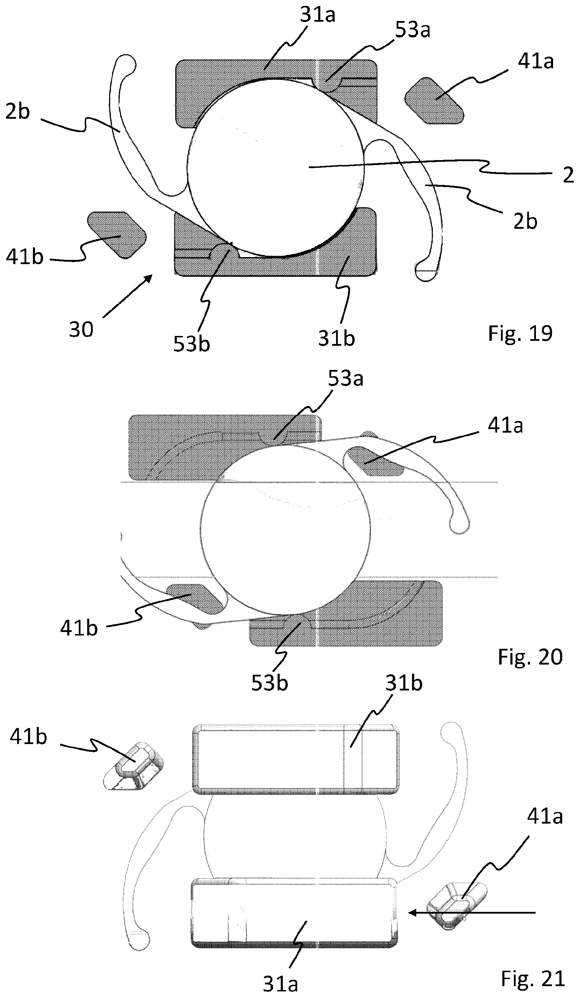

Fig. 19 bis Fig. 21 zeigen schematisch eine Ausführungsform eines Injektors, bei welcher der Haptikschieber in einer Ebene unterhalb der Ebene, in welcher die Linse 2 gehalten ist, angeordnet sein kann.

Fig. 19 und Fig. 20 sind Längsschnitte entlang einer Horizontalebene, wobei Fig. 19 den nicht aktivierten und Fig. 20 den aktivierten Zustand des Haptikschiebers zeigt.

Der Haptikschieber umfasst zwei seitlich gegenüberliegende Schiebeelemente 31a, 31b welche zueinander in Längsrichtung verschiebbar sind.

Bei dieser Ausführungsform ist nur das erste Schiebeelement 31a beweglich. Dies vereinfacht die Ausgestaltung des Injektors.

Zum Einfalten der Haptik umfassen die Schiebeelemente 31a, 31b, ähnlich wie bei dem Ausführungsbeispiel gemäß Fig. 5 bis Fig. 7, jeweils einen Kopf 41a, 41b. Da nur das erste Schiebeelement 31a beweglich ist, muss bei diesem Ausführungsbeispiel die Linse 2 zum Einfalten der Arme der Haptik 2b in Richtung des Kopfes 41b bewegt werden.

Hierzu umfassen die Schiebeelemente 31a, 31b jeweils einen Mitnehmer 53a, 53b.

Der Mitnehmer 53a, 53b liegt seitlich an dem optischen Teil der Linse 2 an.

Bewegt sich nunmehr beim Verschieben des Schiebeelements 31a der Mitnehmer 53a an der Linse 2 entlang, so drückt der Mitnehmer 53a die Linse gegen den Mitnehmer 53b. Die Linse 2 ist elastisch, so dass sich die Mitnehmer 53a, 53b in die Linse 2 einpressen können.

Die Linse 2 wird nunmehr von den Mitnehmern 53a, 53b um einen bauartbedingt vorgegebenen Winkel gedreht und wandert hierdurch in die in Fig. 20 dargestellte Endposition.

Die Köpfe 41a, 41b der Schiebeelemente 31a, 31b falten dabei die Linsenhaptik ein, wobei das Einfalten teilweise auch auf die Drehung der Linse zurückzuführen ist.

Fig. 21 ist eine Draufsicht auf die Unterseite des Magazins.

Zu erkennen ist, dass zwischen den Schiebeelementen 31a, 31b mit den Köpfen 41a, 41b derart viel Raum vorhanden ist, so dass der Durchlass zwischen den Schiebeelementen 31a, 31b hinreichend breit ist, um die gefaltete Linse zwischen den Schiebeelementen 31a, 31b hindurch in den Kanal zu transferieren, über den die Linse appliziert werden kann. Vorzugsweise sind die hier nicht dargestellten Bestandteile des Injektors entsprechend den vorangegangenen Ausführungsbeispielen aufgebaut. Insbesondere kann der Injektor ein Faltelement aufweisen, welches an einer Klappe angeordnet ist, die vor dem Applizieren der Intraokularlinse 2 geschlossen wird.

Durch die Erfindung konnte ein Injektor für Intraokularlinsen bereitgestellt werden, welcher sich leichter bedienen lässt. Insbesondere kann die Aktivierung der Schiebelemente eine Haptikschiebers gekoppelt und/oder das Faltelement gesperrt sein, bis der Haptikschieber aktiviert wurde.

### Bezugszeichenliste

- 1: Injektor
- 2: Intraokularlinse
- 2a: Linsenkörper (optischer Bestandteil)
- 2b: Haptik
- 10: Gehäuse
- 11: Kanüle
- 12: Klappe
- 13: Faltelement
- 14: Lagerbock
- 15: Filmscharnier
- 16: Kanal
- 17: Rasthaken (für hinteren Gehäuseabschnitt)
- 18: Rasthaken (Klappe)
- 19: Schiene
- 20: Anschlussstück für hinteren Gehäuseabschnitt
- 30: Magazin
- 31a: erstes Schiebeelement
- 31b: zweites Schiebeelement (31a + 31b = Haptikschieber)
- 32: Ausnehmung
- 33: Nut
- 34: Wippe
- 35a,35b: Stange
- 36: Drehachse
- 37: Scharnier
- 38: Rasthaken
- 39: weiteres Scharnier
- 40: Schiene
- 41a,41b: Kopf des Schiebeelements
- 42: Halteebene
- 43: Schiene
- 44: Mittelstück
- 45: Kanal
- 46: Schiene
- 47: Kopfstück
- 48: Kolben
- 49: Anlagefläche
- 50: Rasthaken
- 51: Drehteller
- 52: Durchlass
- 53a,53b: Mitnehmer
- 60: Behälter
- 61: Kanal
- 62: Anschlag

## Patentansprüche

1. System, umfassend einen Behälter (60), sowie einen Injektor (1) für eine Intraokularlinse (2), wobei der Injektor (1) einen Haptikschieber zum Einfalten einer Haptik (2b) der Intraokularlinse (2) umfasst, **dadurch gekennzeichnet, dass** der Haptikschieber durch Einbringen des Injektors (1) in den Behälter (60) aktivierbar ist.

2. System nach dem vorstehenden Anspruch, **dadurch gekennzeichnet, dass** der Injektor (1) ein auf den Injektor (1) aufschiebbares Magazin (30) für die Intraokularlinse (2) umfasst, wobei das Magazin (30) in den Behälter (60) einschiebbar ist und den Haptikschieber umfasst.

3. System nach einem der vorstehenden Ansprüche, **dadurch gekennzeichnet, dass** der Behälter (60) als Wärmebehälter zum Erwärmen der Intraokularline (2) ausgebildet ist.

4. System nach einem der vorstehenden Ansprüche, **dadurch gekennzeichnet, dass** der Behälter (60) als ein mit Flüssigkeit gefüllter Behälter (60) zum Lagern und Transportieren einer hydrophilen Intraokularlinse (2) ausgebildet ist.

5. System nach einem der vorstehenden Ansprüche, **dadurch gekennzeichnet, dass** der Haptikschieber durch Einbringen des Magazins (30) nebst Injektor (1) in den Behälter (60) aktivierbar ist.

6. System nach einem der vorstehenden Ansprüche, **dadurch gekennzeichnet, dass** das Magazin (30) und/oder das Gehäuse (10) des Injektors (1) auf einen Anschlag (62) drückbar ist, um den Haptikschieber zu aktivieren.

7. Injektor (1) für eine Intraokularlinse (2), ausgebildet für ein System nach einem der vorstehenden Ansprüche, umfassend ein Gehäuse (10) mit einem im Gehäuse (10) verschiebbaren Kolben zum Applizieren der Intraokularlinse (2), wobei der Injektor (1) ein auf das Gehäuse (10) des Injektors (1) aufschiebbares Magazin (30) für die Intraokularlinse (2) umfasst, **dadurch gekennzeichnet, dass** der Injektor (1) einen Haptikschieber umfasst, welcher durch das Aufschieben des Magazins (30) aktivierbar ist, wenn das Magazin (30) in dem Behälter (60) sitzt und mittels des Gehäuses (10) des Injektors (1) aufgenommen wird.

8. Injektor (1) nach dem vorstehenden Anspruch, **dadurch gekennzeichnet, dass** der Haptikschieber Teil des Magazins (30) ist.

## Claims

1. A system comprising a container (60) and an injector (1) for an intraocular lens (2);
the injector (1) comprising a haptics slider for folding inward haptics (2b) of the intraocular lens (2);
**characterised in that** the haptics slider can be activated by insertion of the injector (1) into the container (60).

2. The system according to the preceding claim, **characterised in that** the injector (1) comprises a cartridge (30) for the intraocular lens (2), which can be slidably fitted onto the injector (1), wherein the cartridge (30) is insertable into the container (60) and comprises the haptics slider.

3. The system according to any one of the preceding claims, **characterised in that** the container (60) is in the form of a heating container for heating the intraocular lens (2).

4. The system according to any one of the preceding claims, **characterised in that** the container (60) is in the form of a liquid-filled container (60) for storing and transporting a hydrophilic intraocular lens (2).

5. The system according to any one of the preceding claims, **characterised in that** the haptics slider can be activated by inserting the cartridge (30) together with the injector (1) into the container (60).

6. The system according to any one of the preceding claims, **characterised in that** the cartridge (30) and/or the housing (10) of the injector (1) can be pressed against a stop (62) in order to activate the haptics slider.

7. An injector (1) for an intraocular lens (2), configured for a system according to any one of the preceding claims, comprising a housing (10) with a plunger that is slideable inside the housing (10) for applying the intraocular lens (2), wherein the injector (1) comprises a cartridge (30) for the intraocular lens (2), which cartridge can be slideably fitted onto the housing (10) of the injector (1);
**characterised in that** the injector (1) comprises a haptics slider which can be activated by the slideable fitting of the cartridge (30) when the cartridge (30) sits in the container (60) and is accommodated by the housing (10) of the injector (1).

8. The injector (1) according to the preceding claim, **characterised in that** the haptics slider forms part of the cartridge (30).

## Revendications

1. Système, comprenant un récipient (60) ainsi qu'un injecteur (1) pour une lentille intraoculaire (2),
l'injecteur (1) comprenant un curseur de haptique destiné au pliage d'une haptique (2b) de la lentille intraoculaire (2),
**caractérisé en ce que** le curseur de haptique est activable par l'introduction de l'injecteur (1) dans le récipient (60).

2. Système selon la revendication précédente, **caractérisé en ce que** l'injecteur (1) comprend une cartouche (30) pour la lentille intraoculaire (2) qui est montable par glissement sur l'injecteur (1), la cartouche (30) étant insérable dans le récipient (60) et comprenant le curseur de haptique.

3. Système selon l'une quelconque des revendications précédentes, **caractérisé en ce que** le récipient (60) est réalisé sous forme de récipient thermique destiné au réchauffement de la lentille intraoculaire (2).

4. Système selon l'une quelconque des revendications précédentes, **caractérisé en ce que** le récipient (60) est réalisé sous forme d'un récipient (60) rempli de liquide pour stocker et transporter une lentille intraoculaire hydrophile (2).

5. Système selon l'une quelconque des revendications précédentes, **caractérisé en ce que** le curseur de haptique est activable par l'introduction de la cartouche (30) conjointement avec l'injecteur (1) dans le récipient (60).

6. Système selon l'une quelconque des revendications précédentes, **caractérisé en ce que** la cartouche (30) et/ou le boîtier (10) de l'injecteur (1) peut être poussé contre une butée (62) afin d'activer le curseur de haptique.

7. Injecteur (1) pour une lentille intraoculaire (2), configuré pour un système selon l'une quelconque des revendications précédentes, comprenant un boîtier (10) avec un piston déplaçable dans le boîtier (10) pour appliquer la lentille intraoculaire (2), l'injecteur (1) comprenant une cartouche (30) pour la lentille intraoculaire (2) qui est montable par glissement sur le boîtier (10) de l'injecteur (1),
**caractérisé en ce que** l'injecteur (1) comprend un curseur de haptique qui est activable par le montage par glissement de la cartouche (30), lorsque la cartouche (30) est disposée dans le récipient (60) et est reçu par le boîtier (10) de l'injecteur (1).

8. Injecteur (1) selon la revendication précédente, **caractérisé en ce que** le curseur de haptique fait partie de la cartouche (30).
